# EUROPEAN PATENT APPLICATION

(11) **EP 3 929 588 A1**
(43) Date of publication of application: **29.12.2021**
(21) Application number: 20901926.4
(22) Date of filing: 21.10.2020
(51) Int. Cl.: G01N 33/68

(54) **APPLICATION OF SERUM PROTEIN HFREP1 IN DIAGNOSIS OF RHEUMATOID ARTHRITIS DISEASE STATE**

(30) Priority: 20.12.2019 CN 201911322787
(71) Applicant: Jiangsu Precise Medical Technology Co., Ltd., Nanjing, Jiangsu 211200 (CN)
(72) Inventor: LIU, Shijia, Nanjing, Jiangsu 210029 (CN); ZHOU, Wei, Nanjing, Jiangsu 210029 (CN); JI, Wei, Nanjing, Jiangsu 210029 (CN); TAN, Wenfeng, Nanjing, Jiangsu 210029 (CN); GUO, Yunke, Nanjing, Jiangsu 210029 (CN); KE, Mengying, Nanjing, Jiangsu 210029 (CN); LU, Jiawei, Nanjing, Jiangsu 210029 (CN)
(74) Representative: ABG Intellectual Property Law, S.L.
(86) International application number: PCT/CN2020/122351
(87) International publication number: WO 2021/120827

(57) **Abstract**

Provided is an application of a serum protein HFREP1 in the diagnosis of rheumatoid arthritis disease state. According to DAS28, a rheumatoid arthritis (RA) disease activity scoring system recommended by the European Anti-Rheumatism Alliance, the RA disease state is divided into an active phase and a remission phase. However, the DAS28 scoring system is a set of subjective scoring system, which requires high doctor experience. The serum protein HFREP1 has an extremely high diagnostic significance in the diagnosis and classification of rheumatoid arthritis in the active and remission phases, has high diagnosis accuracy. Compared with the existing DAS28 scoring system, the serum protein HFREP1 is objective and easy to operate, and can be used to prepare a diagnostic kit for the diagnosis and classification of rheumatoid arthritis in the active and remission phases.

## Description

This application claims the priority of Chinese Patent Application No. 201911322787.2, filed to China National Intellectual Property Administration on December 20, 2019, and titled with "APPLICATION OF SERUM PROTEIN HFREP1 IN DIAGNOSIS OF RHEUMATOID ARTHRITIS DISEASE STATE".

### FIELD

The present disclosure relates to the field of disease diagnosis, involving uses of biomarkers in disease diagnosis, specifically use of serum HFREP1 (also called fibrinogen-like protein 1, FGL1) for diagnosis of disease stage of rheumatoid arthritis.

### BACKGROUND

Rheumatoid arthritis (RA) is a systemic autoimmune disease with erosive arthritis as the main manifestation. This disease has an incidence of 0.2%-0.4% in China, and is more common in women. The pathological manifestation includes chronic inflammation of synovial joints, formation of pannus, and destruction of the cartilage and bone of joints, which will eventually lead to joint deformity and loss of function. The current diagnosis of RA is based on the standards of American College of Rheumatology (ACR) and European League against Rheumatism (EULAR). EULAR recommends the disease stage score in 28 joints (DAS28) for RA. According to this scoring system, the stage of RA is classified into activity stage and remission stage.

However, DAS28 scoring system is a subjective scoring system, which requires doctors of rich experience.

In order to improve the objectivity of the diagnosis of RA stage, the present invention is completed.

### SUMMARY

In order to overcome the shortcomings of the prior art, the present disclosure provides use of serum HFREP1 (hepatocyte-derived fibrinogen-related protein 1) for diagnosis of disease stage of rheumatoid arthritis.

The present disclosure provides the following technical solutions.

Use of serum HFREP1 for the manufacture of a reagent or kit for diagnosis of stage of rheumatoid arthritis, wherein the stage of rheumatoid arthritis is activity stage or remission stage.

In the test set, when serum HFREP1 is used to diagnose and classify activity stage and remission stage in patients with rheumatoid arthritis, the ROC curve has an area under the curve (AUC) as high as 0.9843, a sensitivity of 0.9412, and a specificity of 0.9667. In the validation set, when serum HFREP1 is used to diagnose and classify activity stage and remission stage in patients with rheumatoid arthritis, the accuracy serum HFREPlis as high as 97.62%. It can be seen that the serum HFREP1 has extremely high diagnostic value in diagnosing and classifying the activity and remission stages of rheumatoid arthritis. Due to its high diagnostic accuracy and advantages of being more objective and easier to operate than the existing DAS28 scoring system, the serum HFREP1 can be used to prepare a diagnostic kit for diagnosing and classifying the activity and remission stages of rheumatoid arthritis.

### BRIEF DESCRIPTION OF DRAWINGS

FIG. 1 shows the ROC curve of using HFREP1 to diagnose and classify activity stage and remission stage of the patients with rheumatoid arthritis in the test set.
FIG. 2 shows the sample distribution of using HFREP1 to diagnose and classify activity stage and remission stage of the patients with rheumatoid arthritis in the validation set.

### DETAILED DESCRIPTION

The essential content of the present disclosure is described in detail with reference to the accompanying drawings and examples, but the protection scope of the present disclosure is not limited thereto.

### Example 1

### HFREP1 in Diagnosis and Classification of Disease stage of Patients with Rheumatoid Arthritis

### 1. Experimental samples and reagents

The sample in the test set: samples from 34 patients with rheumatoid arthritis in remission stage and 30 patients with rheumatoid arthritis in activity stage were collected from Jiangsu Province Hospital of Chinese Medicine. There were no significant differences in age, gender and body mass index between patients in remission stage and in activity stage.

The sample in the validation set: samples from 26 patients with rheumatoid arthritis in remission stage and 16 patients with rheumatoid arthritis in activity stage were collected from Jiangsu Province Hospital. There were no significant differences in age, gender and body mass index between patients in remission stage and in activity stage.

The enrollment criteria for patients with rheumatoid arthritis in remission stage and in activity stage are as follows:

2010 ACR-EULAR Classification Criteria for Rheumatoid Arthritis

| | 0 point | 1 point | 2 points | 3 points | 5 points |
|---|---|---|---|---|---|
| Joint involvement | 1 medium/large | 2-10 medium/large | 1-3 small | 4-10 small | >10 |
| Serology antibody test | Negative RF and negative anti-CCP | | Low-positive RF or low-positive anti-CCP | High-positive RF or high-positive anti-CCP | |
| Duration of synovitis | <6 weeks | ≥6 weeks | | | |
| Acute-phase reactants | Normal CRP and normal ESR | Elevated CRP or elevated ESR | | | |

Note: The highest score is selected for each part. For example, if a patient has 5 small joints and 4 large joints involved, 3 points are awarded. If the sum of the scores of the four parts is ≥6 points, the patient is diagnosed with RA. Distal interphalangeal joints, first carpometacarpal joints, and first metatarsophalangeal joints are excluded from joint involvement. Medium/large joints: shoulders, elbows, hips, knees, and ankles. Small joints: the other joints except for the medium/large joints and excluding distal interphalangeal joints, first carpometacarpal joints, and first metatarsophalangeal joints. Low titer is defined as exceeding the upper limit of normal, but not higher than 3 times the upper limit of normal. High titer is defined as exceeding 3 times the upper limit of normal.

The criteria for determining the disease stage of rheumatoid arthritis (using the disease stage score in 28 joints (DAS28)): DAS28<2.6 is the remission stage, and DAS28>2.6 is the activity stage.

Exclusion criteria: (1) Those who do not meet the diagnostic criteria for rheumatoid arthritis and related diagnostic criteria for the activity stage of RA. (2) Those who have taken western medicine or other drugs for treatment for a long time and cannot withdrawal. (3) Those who have other rheumatism, such as systemic lupus erythematosus, Sjogren syndrome, severe osteoarthritis, etc. (4) Those with severe deformity or loss of labor. (5) Those with severe cardiopulmonary function or liver, kidney and hematopoietic system diseases. (6) Women who are pregnant or breastfeeding. (7) Those who himself or whose family member is unwilling to participate in clinical trials. (8) Those who suffer from mental illness and cannot communicate normally.

Weeding criteria: (1) Those with poor compliance. (2)Those who did not take the study drug as prescribed by the doctor after enrolled. (3) Those who did not have any evaluable data after enrolled.

Drop-out criteria: (1) Those who had severe adverse events or complications and are no longer suitable for continuing the experiment. (2) Those who drop off or lose to follow-up during the experiment. Control the drop-out subjects in the cohort less than 10%.

Experimental reagents:

HFREP1 ELISA kit was purchased from RayBiotech., Inc.

### 2. Experimental method

### (1) Collection and storage of serum samples

Peripheral blood samples were collected from patients after fasting in the morning and placed in a tube without anticoagulant. The blood sample was then left at room temperature for 30-60 min to agglutinate naturally. Upon agglutination, the blood sample was subjected to centrifugation at 2000 rpm for 10 min, and the upper clear serum was carefully transferred to a sterile lyophilization tube, then labeled and placed in a -80°C refrigerator for later use.

### (2) Determining the amount of target protein in serum by ELISA

The amount of HFREP1 in serum was determined by the ELISA kit following the instructions.

### (3) Data processing

The ROC curve (receiver operating characteristic curve) of target protein in the test set was established, of which the area under the curve (AUC) and 95% confidence interval were calculated. Logistic regression was employed to establish a regression equation to generate a set of new variables logit[P]. After conversion of logit[P]=Ln^{[p/(1-p)]}, the predicted probability p was obtained, and the ROC curve analysis was performed on this new variable. In the validation set, the best cut-off value obtained by the ROC curve was used as a threshold to calculate the diagnostic accuracy of using the target protein for the disease stage of rheumatoid arthritis.

### 3. Experimental results

### (1) The different abundance of target protein in the stages of rheumatoid arthritis

In the training set, the absolute amount of serum HFREP1 in patients with rheumatoid arthritis in activity stage was significantly increased compared with patients in remission stage. The detection results are shown in the following table.

| Group | HFREP1 amount (µg/mL) |
|---|---|
| Patients with rheumatoid arthritis in activity stage | 10.31±16.47 |
| Patients with rheumatoid arthritis in remission stage | 0.60±0.42 |

### (2) ROC curve of the target protein for diagnosis and classification activity stage and remission stage in patients with rheumatoid arthritis

### Principle of ROC curve for evaluation

For the diagnostic test, the basic evaluation indicators include sensitivity, specificity, etc., and the comprehensive evaluation indicators include Youden index, ROC, AUC etc. For the evaluation of a diagnostic test, it is necessary to know the true property of the sample to be tested by the "gold standard". Based on the groups determined by the gold standard, that is, patients with rheumatoid arthritis in activity stage or in remission stage, the results of the diagnostic test can be divided into the following situations:
True Positive (TP): positive result determined by the diagnostic test (consistent with the gold standard result).
True Negative (TN): negative result determined by the diagnostic test (consistent with the gold standard result).
False Positive (FP): positive result determined by the diagnostic test (inconsistent with the gold standard result).
False Negative (FN): negative result determined by the diagnostic test (inconsistent with the gold standard result).

It can be represented by the following table:

| Diagnostic test | Gold standard | | Total |
|---|---|---|---|
| | Rheumatoid arthritis in remission stage | Rheumatoid arthritis in activity stage | |
| Positive | A ( TP numbers ) | B ( FP numbers ) | A+B |
| Negative | C ( FN numbers ) | D ( TN numbers ) | C+D |
| Total | A+C | B+D | / |

The sensitivity of the diagnostic test = A/(A+C), and the specificity = D/(B+D). Through the sensitivity and specificity, the diagnostic sensitivity and specificity of the diagnostic test relative to the gold standard can be obtained. High sensitivity means fewer cases of rheumatoid arthritis in remission stage were diagnosed as negative, and a low missed diagnosis rate. High specificity means fewer cases of rheumatoid arthritis in activity stage were diagnosed as positive, and a low misdiagnosis rate.

The ROC curve is plotted based on the above-mentioned sensitivity and specificity. The possible diagnostic threshold in the diagnostic test was used as a diagnostic point, and the corresponding sensitivity and specificity were calculated according to the above table. Then, sensitivity was used as the ordinate and 1 -specificity as the abscissa. The sensitivity and specificity at each diagnosis point were marked in the coordinate chart, and the coordinate points were connected to obtain a smooth curve, which is the ROC curve. The more the diagnosis points are set, the smoother the ROC curve obtained.

The ROC curve uses each test result as a possible diagnostic threshold, and the size of the area under the curve (AUC) indicates the accuracy of the diagnostic test. The area under the curve (AUC) of the ROC curve has been generally recognized as an inherent accuracy indicator for the authenticity evaluation of diagnostic tests. When AUC is 0.5, there is no diagnostic significance. When AUC is 0.5 to 0.7, the diagnostic accuracy is low. When AUC is 0.7 to 0.9, the diagnosis accuracy is medium. When AUC>0.9, the diagnosis has high accuracy.

### (3) HFREP1 was used to diagnose and classify activity stage and remission stage in patients with rheumatoid arthritis

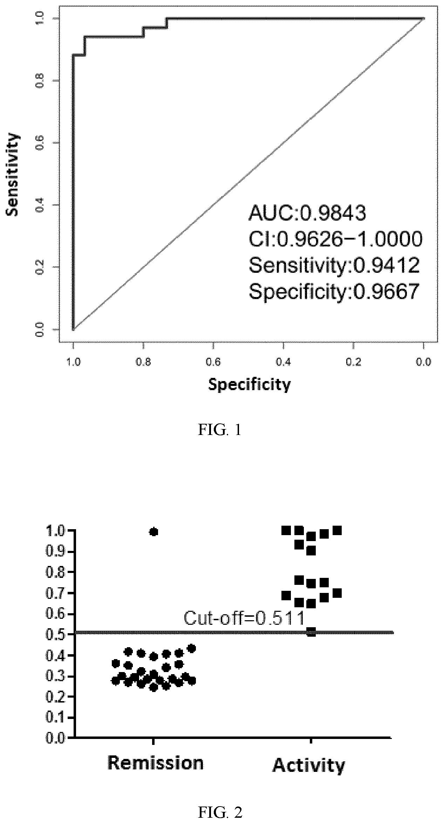

The amount of HFREP1 protein (X) in the training set sample was used as the independent variable, and the group (i.e., patients with rheumatoid arthritis in remission stage or in activity stage) was used as the dependent variable. Binary logistic regression was performed on the amount of target protein in the serum samples in patients with rheumatoid arthritis in remission stage and in activity stage, and the binary logistic regression equation was generated: Ln^{[p/(1-p)]}=3.160X-4.809. Then the absolute amount of the target protein in each serum sample was substituted into the binary logistic regression equation to obtain the regression prediction probability p of each serum sample. The possible regression prediction probability p was used as a diagnosis point to calculate the sensitivity and specificity, based on which the ROC curve was plotted (as shown in FIG. 1) with AUC of 0.9843, a sensitivity of 0.9412, and a specificity of 0.9667. According to the coordinates of the ROC curve, Youden index was calculated, where Youden index = specificity + sensitivity - 1. The corresponding predicted probability p value of the maximum Youden index is the best cut-off value of 0.511 to diagnose and classify remission stage and activity stage in patients with rheumatoid arthritis.

### (4) Verification of the accuracy of using the target protein to diagnose and classify remission stage and activity stage in patients with rheumatoid arthritis

In the validation set, the best cut-off value, obtained by using HFREP1, was used as the diagnostic threshold to predict the serum samples from patients with rheumatoid arthritis in remission stage or in activity stage. The number of correctly predicted samples divided by the total number of samples (26+16=42) is the accuracy of using the target protein to diagnose and classify between patients with rheumatoid arthritis at remission stage vs. at activity stage. The accuracy of using HFREP1 to diagnose and classify remission stage and activity stage in patients with rheumatoid arthritis was 97.62%, and the sample distribution is shown in FIG. 2.

In summary, the HFREP1 level in serum has extremely high diagnostic value in diagnosing and classifying remission stage and activity stage in patients with rheumatoid arthritis. Due to its high diagnostic accuracy and advantages of being more objective and easier to operate than the existing DAS28 scoring system, the serum HFREP1 can be used to prepare a diagnostic kit for diagnosing and classifying remission stage and activity stage in patients with rheumatoid arthritis.

### Example 2

### Kit for Diagnosing and Classifying Activity and Remission of Rheumatoid Arthritis

A kit for diagnosing and classifying activity and remission of rheumatoid arthritis comprises ELISA reagents for determining the amount of serum HFREP1.

The foregoing examples mentioned above are to specifically introduce the essential content of the present disclosure, but it will be understood by those skilled in the art that the protection scope of the present disclosure should not be limited to these specific examples.

## Claims

1. Use of serum HFREP1 for the manufacture of a reagent or kit for diagnosis of disease stage of rheumatoid arthritis, wherein the disease stage of rheumatoid arthritis is activity stage or remission stage.
